# EUROPÄISCHE PATENTSCHRIFT

(11) **EP 1 739 432 B1**
(45) Veröffentlichungstag und Bekanntmachung des Hinweises auf die Patenterteilung: **12.10.2011**
(21) Anmeldenummer: 05014014.4
(22) Anmeldetag: 29.06.2005
(51) Int. Cl.: G01N 35/00, G01N 33/487

(54) **Analysesystem mit Testband**
Analysis system with test band
Système d'analyse avec bande de test

(43) Veröffentlichungstag der Anmeldung: 03.01.2007
(73) Patentinhaber: F. Hoffmann-La Roche AG, 4070 Basel (CH); Roche Diagnostics GmbH, 68305 Mannheim (DE)
(72) Erfinder: Seidenstricker, Manfred, 68259 Mannheim (DE); Miltner, Karl, 67227 Frankenthal (DE); Baumann, Edgar, 68219 Mannheim (DE); Bogumil, Konstanze, 68169 Mannheim (DE); Heck, Wolfgang, D-68526 Ladenburg (DE)
(74) Vertreter: Pfiz, Thomas

(56) Entgegenhaltungen:
- EP-A- 0 371 572
- EP-A- 1 304 162
- WO-A-2005/047861
- US-A- 4 204 767
- US-A1- 2002 041 829

## Beschreibung

Die Erfindung betrifft ein Analysesystem zur Verarbeitung eines Testbands, zur Untersuchung von Körperflüssigkeiten mit einem flexiblen Trägerband und einer Vielzahl von darauf im Abstand voneinander angeordneten Testfeldern für den Nachweis eines Analyten in der Körperflüssigkeit.

Derartige Testbänder lassen sich zum Nachweis von Inhaltsstoffen in einer Körperflüssigkeit wie Blut oder Urin in automatisch arbeitenden Handgeräten einsetzen, mit denen auch von Laien die erforderlichen Untersuchungsschritte einfach und schnell vorgenommen werden können. Anstelle herkömmlicher einzelner Teststreifen sind auf dem aufgewickelten Testband eine Vielzahl von mit einer geeigneten Testchemie versehenen Testfeldern fortlaufend angeordnet. Die Körperflüssigkeit wird dabei auf einem durch Bandvorlauf in eine aktive Position gebrachten Testfeld appliziert, um sodann einen Nachweis beispielsweise durch eine optische Untersuchung zu ermöglichen. Dabei sollen auf dem Bandtransportweg auftretende Momentenschwankungen der mechanischen Last durch Reibung, wachsenden Durchmesser des Bandwickels auf der Zugspule und daraus resultierende Geschwindigkeitsänderungen keinen Einfluss auf die exakte Positionierung der Testfelder am Applikations- bzw. Messort haben. Soweit hier Positionierantriebe mit Positionsgebern an ihren Wellen oder angekoppelten Maschinenteilen eingesetzt werden, können mechanische Toleranzen von Bauelementen, Durchmesservariationen der Bandwickel und Bandschlupf zu Abweichungen von den gewünschten Sollpositionen führen.

In der DE 103 43 896 A1 der Anmelderin sind verschiedene Funktionsfelder neben den Testfeldern für ein Testband offenbart, insbesondere zur Benutzerinformation oder für spezielle Gerätefunktionen.

Die WO 2005/047861 offenbart ein tragbares Blutzuckermessgerät mit Bandkassette zur Aufnahme eines Testbands, auf welchem Führungslöcher für den mechanischen Eingriff von Sprossen auf der Aufwickelspule zur Verhinderung eines Bandschlupfs vorgesehen sind.

Ausgehend hiervon liegt der Erfindung die Aufgabe zugrunde, weitere Verbesserungen gegenüber dem Stand der Technik zu schaffen und die Funktionalität der gattungsgemäßen Erzeugnisse vor allem hinsichtlich der Positioniergenauigkeit zu optimieren.

Zur Lösung dieser Aufgabe wird die in dem unabhängigen Patentanspruch angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine Wegmessung direkt auf dem eigentlich zu positionierenden Objekt zu ermöglichen. Dementsprechend wird erfindungsgemäß vorgeschlagen, dass das Trägerband mit Markierungsbereichen zur Wegerfassung beim Bandtransport versehen ist. Auf diese Weise kann unabhängig vom Bandantrieb eine unmittelbare Lagebestimmung der Testfelder und etwaiger zusätzlicher Funktionsabschnitte vorgenommen werden, so dass eine exakte und schnelle Positionierung möglich ist. Dies erlaubt es, die Testfeldlänge gering zu halten und somit auch den Raumbedarf für das Testband zu reduzieren. Auch das erforderliche Volumen der aufzutragenden Körperflüssigkeit kann bei exakter Applikation und lagerichtiger Vermessung weiter verringert werden. Ein weiterer Vorteil ergibt sich bereits bei der Bandherstellung für die genaue Durchführung von Fertigungsprozessen wie Schneiden und Kleben.

Vorteilhafterweise erstrecken sich die Markierungsbereiche jeweils über einen Bandabschnitt zwischen aufeinander folgenden Testfeldern in Bandrichtung. Grundsätzlich ist es aber auch möglich, dass die Markierungsbereiche kontinuierlich entlang dem Band etwa auf dessen Rückseite oder Rand verlaufen.

Eine weitere vorteilhafte Ausführung sieht vor, dass die Markierungsbereiche einen inkremental abtastbaren Wegmaßstab bilden. Dies lässt sich einfach dadurch realisieren, dass die Markierungsbereiche durch vorzugsweise aus alternierend hellen und dunklen Segmenten bestehende Rasterstreifen gebildet sind. In einer weiteren günstigen Ausführung bestehen die Segmente aus Metall. Diese können elektrisch oder magnetisch abgetastet werden.

Um auch eine Information über die Bewegungsrichtung beim Bandtransport zu gewinnen, ist es vorteilhaft, wenn die Markierungsbereiche zwei parallel zueinander verlaufende, in Bandrichtung gegeneinander versetzte und damit mit richtungsabhängiger Phasenverschiebung abtastbare Rasterstreifen aufweisen.

Denkbar ist es auch dass die Markierungsbereiche einen absolut codierten Wegmaßstab beispielsweise im Gray-Code aufweisen.

Um eine möglichst effektive Antriebsregelung zu erlauben, ist es von Vorteil, wenn die Wegerfassungseinrichtung die Bewegungsrichtung und den beim Bandtransport zurückgelegten Weg direkt an dem Testband erfasst.

Eine weitere bevorzugte Ausgestaltung sieht vor, dass die Wegerfassungseinrichtung in Bandtransportrichtung gesehen in einem vorgegebenen Abstand von einer die Testfelder abtastenden Messeinheit angeordnet ist.

Für ein selbsttaktendes Auslesen ist es vorteilhaft, dass die Wegerfassungseinrichtung seitlich an dem Testband positionierte Sensormittel zur vorzugsweise berührungslosen Abtastung der Markierungsbereiche beim Bandtransport aufweist.

Eine besonders bevorzugte Ausführung sieht vor, dass die Bandtransportvorrichtung eine mit der Wegerfassungseinrichtung gekoppelte Antriebsregelung für den Transport des Testbands in vorgegebene Sollpositionen aufweist. Auf diese Weise ist ein rasches und genaues Anfahren der Zielposition möglich, so dass auch die Wartezeiten für den Benutzer reduziert werden.

Um ein robustes Bremsen weiter zu unterstützen, ist es günstig, wenn die Bandtransportvorrichtung eine in Abhängigkeit von einem Ausgangssignal der Wegerfassungseinrichtung gegenläufig zur Bandzugrichtung angesteuerte Antriebseinheit umfasst. Durch das kurzzeitige Anlegen einer elektrischen Gegenspannung, d.h. Umkehrung der Spannung mit geeigneter Spannungshöhe, an die Antriebseinheit kann die Bandbewegung bis zum Stillstand aktiv und robust gebremst werden.

Im Folgenden wird die Erfindung anhand eines in der Zeichnung schematisch dargestellten Ausführungsbeispiels näher erläutert. Es zeigen
- Fig. 1: ein Blockschaltbild eines Analysesystems zur Verarbeitung eines Testbands mit Wegmarkierungen;
- Fig. 2: das Analysesystem als Handgerät in vereinfachter Schnittdarstellung.

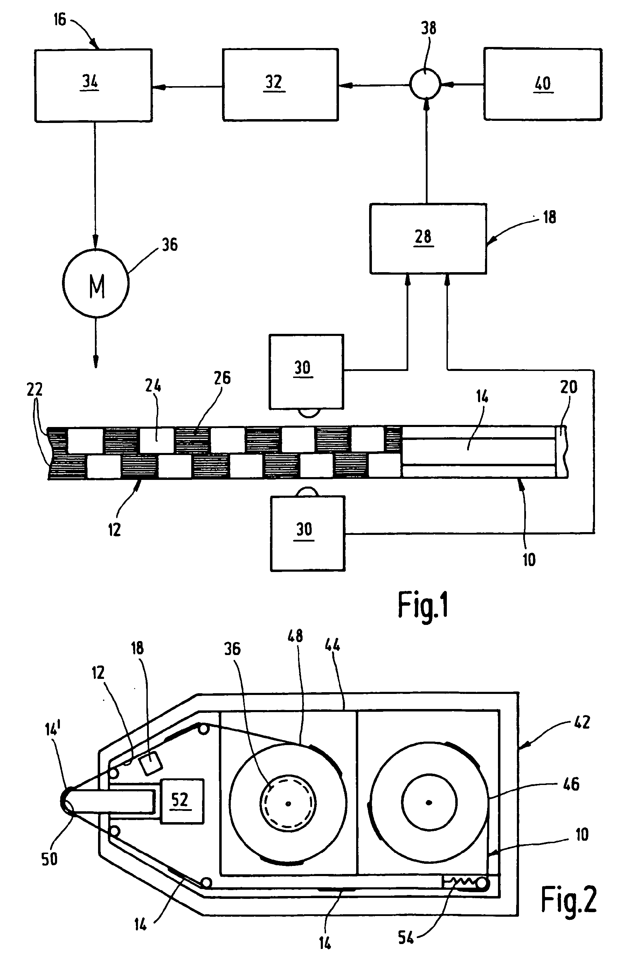

Das Fig. 1 gezeigte Analysesystem umfasst ein Testband 10 mit Wegmarkierungsbereichen 12 und Testfeldern 14, eine Bandtransportvorrichtung 16 zum sukzessiven Positionieren der Testfelder in einer aktiven Position und eine die Markierungsbereiche 12 abtastende Wegerfassungseinrichtung 18 zur Ansteuerung der Bandtransportvorrichtung 16 nach Maßgabe der erfassten momentanen Bandposition.

Wie in Fig. 1 ausschnittsweise gezeigt, weist das Testband 10 eine kontinuierliche Trägerfolie 20 auf, welche eine Vielzahl von in Bandrichtung im Abstand voneinander angeordneten Testfeldern 14 trägt. Die Testfelder 14 sind mit Trockenreagenzien für den Nachweis eines Inhaltsstoffs bzw. Analyten in einer aufgebrachten Körperflüssigkeit versehen. Insbesondere ist ein Blutzuckernachweis in applizierter Blutflüssigkeit vorgesehen.

Die Markierungsbereiche 12 sind in den Bandabschnitten zwischen den Testfeldern 14 auf die Trägerfolie 20 aufgebracht. In dem gezeigten Ausführungsbeispiel sind dort zwei parallel zueinander verlaufende, längs des Bandes gegeneinander versetzte Rasterstreifen 22 vorhanden. Diese bestehen aus alternierend hellen/transparenten und dunklen Segmenten 24, 26 und bilden gegeneinander phasenverschoben abtastbare Wegmaßstäbe, wie es weiter unten näher erläutert wird.

Die Wegerfassungseinrichtung 18 weist eine Signalverarbeitungsstufe 28 auf, welche eingangsseitig mit optoelektronischen Sensoren 30 zusammenwirkt. Die breitseitig an dem Testband 10 positionierten Sensoren 30 tasten die einzeln zugeordneten Rasterstreifen 22 nach Art von Lichtschranken ab, so dass beim Bandtransport periodisch elektrische Impulse an einen Zähler der Signalverarbeitungsstufe 28 abgegeben werden. Aufgrund des Streifenversatzes werden dabei die Zählimpulse mit 90° Phasenverschiebung registriert, wodurch neben dem zurückgelegten Weg auch die Bewegungsrichtung unmittelbar an dem Band 10 erfasst wird.

Die Bandtransportvorrichtung 16 umfasst einen Regler 32 mit Stellglied 34 zur Ansteuerung des Positionierantriebs 36 für das Testband 10. Der Regler 32 ist über den Vergleicher 38 mit der Differenz aus einer Wegvorgabe 40 und der Ausgangsgröße der Wegerfassungseinrichtung 18 beaufschlagbar. Damit lässt sich eine schnelle und exakte Positionierung eines Testfelds 14 an der vorgesehenen Messstelle erreichen.

Die Positionserfassung erfolgt also dadurch, dass während der Bandbewegung die Weginkremente bzw. Segmente 24,26 an den Sensoren 30 vorbeibewegt werden, wobei die Flankenwechsel mittels der Erfassungseinrichtung 18 erfasst werden. Die Segmente 24,26 in den Streifen 22 haben eine gleich bleibende Länge bzw. einen vorgegebenen Abstand voneinander in Bandrichtung gesehen. Die Flankenwechsel werden von dem Zähler der Signalverarbeitungsstufe 28 aufsummiert. Der Zählerstand ist daher direkt proportional zum zurückgelegten Weg des Bandes 10.

Die Wegvorgabe 40 gibt in zeitlicher Abfolge Zählervorgaben auf den Vergleicher 38. Dieser bildet aus den Zählervorgaben und dem Zählerstand der Signalverarbeitungsstufe 28 die Differenz. Diese Differenz gibt dem Regler 32 das Maß für die Ansteuerung des Antriebs vor.

Die Trägerfolie 20 kann für einen gewissen Bandvorlauf ohne Rasterstreifen z.B. weiß bedruckt sein. Wird das Band 10 mit dieser Weißzone an den Sensoren 30 vorbeibewegt, so dass diese ein erstes schwarzes Segment 26, ist dies die Referenz für eine absolute Startposition, die der Einrichtung 18 somit bekannt ist und ab der wie oben beschrieben Inkremente gezählt werden.

Fig. 2 veranschaulicht die Verarbeitung eines solchen Testbands 10 in einem Kassettengerät 42 als tragbares Analysesystem. Die Bandkassette 10 weist eine Vorratsspule 46 für unverbrauchtes Testband und eine Abzugspule 48 mit Antrieb 36 für verbrauchtes Testband auf. Die Testfelder 14 können an einer Umlenkspitze 50 mit Probenflüssigkeit bzw. Blut beaufschlagt werden. Der Nachweis des Analyten (Glucose) erfolgt durch reflexionsphotometrische Messung mittels der Messeinheit 52. Dabei ist die Messeinheit 52 über die als Lichtleiter ausgebildete Führungsspitze 50 und durch die transparente Trägerfolie 20 hindurch an ein aktives, d.h. in der Aufnahmeposition befindliches Testfeld 14' optisch angekoppelt. Durch entsprechenden Bandvorlauf können die Testfelder 14 sukzessive zum Einsatz gebracht werden. Auf diese Weise lassen sich Vielfachtests für eine Patienten-Selbstkontrolle durchführen, ohne dass Verbrauchsmittel ausgetauscht werden müssten.

Die Position eines Testfelds 14 an der Führungsspitze 50 als Messort wird also über eine davon um einen definierten Versatz bzw. Weg-Offset entfernte Wegerfassungseinrichtung 18 ermittelt. Wenn das Band 10 bewegt wird und an der Erfassungseinrichtung 18 eine vorgegebene Anzahl von Weginkrementen 24,26 erfasst wurden, so ist unter Berücksichtigung des Weg-Offsets auch das momentan aktive Testfeld 14' an der Zielposition über der optischen Messeinheit 52 positioniert. Der Weg-Offset erlaubt dabei unter optischen (Lichtabschottung) und mechanischen Gesichtspunkten (begrenzter Bauraum im Bereich der Spitze 50) an kritischer Stelle eine Entzerrung von Gerätefunktionen. Zudem ist es mittels der Wegerfassungseinrichtung 18 möglich, während der Messung auf dem Testfeld 14' zugleich die Position des Bandes 10 zu überwachen.

Der Bandvorlauf kann aufgrund des flexiblen Trägerbandes 20 nur unter Zug erfolgen, in der in Fig. 2 gezeigten Ausführung also durch Drehung der Abzugspule 48 im Uhrzeigersinn. Für ein rasches aktives Abbremsen vor Erreichen der Aufnahmeposition kann der Spulenantrieb 36 jedoch kurzfristig mit einem gegenläufigen Antriebsmoment, d.h. hohem Bremsmoment betrieben werden. Dies erfolgt durch Anlegen einer geregelten Gegenspannung mittels der Regeleinrichtung 32,34, wobei ein federbelasteter Bandspanner 54 auf Seite der Vorratsspule 46 für den notwendigen Bandrückzug sorgt. Die Wegerfassungseinrichtung 18 erkennt aufgrund der Wegmarkierungen 12 auch eine eventuell durch zu hohes gegenläufiges Antriebsmoment bedingte Richtungsumkehr der Bandbewegung und schaltet rechtzeitig den Antrieb ab, so dass das Positionsziel ohne Überschwingen erreicht wird. Grundsätzlich ist es auch möglich, dass bei Überfahren des Positionsziels eine erforderliche Bandstrecke wieder zurückgefahren wird.

## Patentansprüche

1. Analysesystem als Handgerät zur Untersuchung von Körperflüssigkeiten mit einem in einer Bandkassette (44) magazinierten Testband, das aus einem flexiblen Trägerband (20) und einer Vielzahl von darauf im Abstand voneinander angeordneten Testfeldern (14) für den Nachweis eines Analyten in der Körperflüssigkeit besteht, und einer Bandtransportvorrichtung (16) zum Positionieren der Testfelder (14), **gekennzeichnet durch** eine Wegerfassungseinrichtung (18) umfassend berührungslos abtastbare Markierungsbereiche (12) auf dem Testband (10) zur Wegerfassung beim Bandtransport und ein seitlich an dem Testband (10) positioniertes Sensormittel (30) zur berührungslosen Abtastung der Markierungsbereiche (12) beim Bandtransport, wobei die Markierungsbereiche (12) sich jeweils über einen Bandabschnitt zwischen aufeinander folgenden Testfeldern (14) in Bandrichtung erstrecken.

2. Analysesystem nach Anspruch 1, **dadurch gekennzeichnet, dass** die Wegerfassungseinrichtung (18) eingerichtet ist, die Bewegungsrichtung und den beim Bandtransport zurückgelegten Weg direkt an dem Testband (10) zu erfassen.

3. Analysesystem nach Anspruch 1 oder 2, **dadurch gekennzeichnet, dass** die Wegerfassungseinrichtung (18) in Bandtransportrichtung gesehen in einem vorgegebenen Abstand von einer die Testfelder (14) abtastenden Messeinheit (52) angeordnet ist.

4. Analysesystem nach einem der Ansprüche 1 bis 3, **dadurch gekennzeichnet, dass** die Bandtransportvorrichtung (16) eine mit der Wegerfassungseinrichtung (18) gekoppelte Arltriebsregelung (32,34) für den Transport des Testbands (10) in vorgegebene Sollpositionen aufweist.

5. Analysesystem nach einem der Ansprüche 1 bis 4, **dadurch gekennzeichnet, dass** die Bandtransportvorrichtung (16) eine in Abhängigkeit von einem Ausgangssignal der Wegerfassungseinrichtung (18) gegenläufig zur Bandvorlaufrichtung ansteuerbare Antriebseinheit (36) umfasst.

6. Analysesystem nach einem der Ansprüche 1 bis 5, **dadurch gekennzeichnet, dass** die Markierungsbereiche (12) einen inkremental abtastbaren Wegmaßstab bilden.

7. Analysesystem nach einem der Ansprüche 1 bis 6, **dadurch gekennzeichnet, dass** die Markierungsbereiche (12) durch vorzugsweise aus alternierend hellen und dunklen Segmenten bestehende Rasterstreifen (22) gebildet sind.

8. Analysesystem nach einem der Ansprüche 1 bis 7, **dadurch gekennzeichnet, dass** die Markierungsbereiche (12) zwei parallel zueinander verlaufende, in Bandrichtung gegeneinander versetzte Rasterstreifen (22) aufweisen.

9. Analysesystem nach einem der Ansprüche 1 bis 5, **dadurch gekennzeichnet, dass** die Markierungsbereiche (12) einen absolut codierten Wegmaßstab aufweisen.

## Claims

1. Analytical system as hand held device for analysing body fluids comprising a test tape (10) stored in a tape cassette (44) and consisting of a flexible carrier tape (20) and a plurality of test fields (14) arranged spaced apart thereon for the detection of an analyte in the body, and comprising a tape transport device (16) for positioning the test fields (14), **characterized by** a distance registering device (18) comprising marker areas (12) on the test tape (10) which can be scanned in a contact-free manner to register the distance during tape transport and a sensor means (30) positioned at the side of the test tape (10) for contact-free scanning the marker areas (12) during tape transport, wherein the marker areas (12) each extend over a tape section between consecutive test fields (14) in the tape direction.

2. Analytical system according to claim 1, **characterized in that** the distance registering device (18) is arranged to detect the direction of movement and the distance travelled during tape transport directly on the test tape (10).

3. Analytical system according to claim 1 or 2, **characterized in that** the distance registering device (18) is located at a predetermined distance in the direction of tape transport from a measuring unit (52) that scans the test fields (14).

4. Analytical system according to one of the claims 1 to 3, **characterized in that** the tape transport device (16) has a drive control (32, 34) for transporting the test tape (10) into predetermined target positions that is coupled to the distance registering device (18).

5. Analytical system according to one of the claims 1 to 4, **characterized in that** the tape transport device (16) comprises a drive unit (36) that can be actuated oppositely directed to the tape advance direction as a function of an output signal of the distance registering device (18).

6. Analytical system according to one of the claims 1 to 5, **characterized in that** the marker areas (12) form a distance scale that can be scanned incrementally.

7. Analytical system according to one of the claims 1 to 6, **characterized in that** the marker areas (12) are formed by raster strips (22) preferably consisting of alternate light and dark segments.

8. Analytical system according to one of the claims 1 to 7, **characterized in that** the marker areas (12) have two parallel raster strips (22) that arc displaced relative to one another in the tape direction.

9. Analytical system according to one of the claims 1 to 5, **characterized in that** the marker areas (12) have an absolutely coded distance scale.

## Revendications

1. Système d'analyse sous forme d'appareil portatif pour examiner des liquides corporels, avec une bande de test stockée dans une cassette de bande (44) et qui est composée d'une bande support flexible (20) et d'une pluralité de zones de test (14) disposées sur celle-ci à distance les unes des autres pour détecter un analyte dans le liquide corporel, et un dispositif de transport de bande (16) pour positionner les zones de test (14), **caractérisé par** un dispositif de détection de déplacement (18) comprenant des zones de marquage (12) explorables sans contact sur la bande de test (10) pour détecter le déplacement pendant le transport de la bande, et un moyen formant capteur (30) positionné sur le côté de la bande de test (10) pour explorer sans contact les zones de marquage (12) pendant le transport de la bande, lesdites zones de marquage (12) s'étendant à chaque fois sur une portion de bande entre des zones de test (14) successives dans le sens de la bande.

2. Système d'analyse selon la revendication 1, **caractérisé en ce que** le dispositif de détection de déplacement (18) est configuré pour détecter directement sur la bande de test (10) le sens de mouvement et le chemin parcouru pendant le transport de la bande.

3. Système d'analyse selon la revendication 1 ou 2, **caractérisé en ce que** le dispositif de détection de déplacement (18), vu dans le sens de transport de la bande, est disposé à une distance prédéfinie d'une unité de mesure (52) explorant les zones de test (14).

4. Système d'analyse selon l'une des revendications 1 à 3, **caractérisé en ce que** le dispositif de transport de bande (16) comporte un réglage d'entraînement (32, 34) couplé avec le dispositif de détection de déplacement (18) pour le transport de la bande de test (10) dans des positions de consigne prédéfinies.

5. Système d'analyse selon l'une des revendications 1 à 4, **caractérisé en ce que** le dispositif de transport de bande (16) comporte une unité d'entraînement (36) apte à être commandée dans le sens contraire du sens d'avancement de la bande en fonction d'un signal de sortie du dispositif de détection de déplacement (18).

6. Système d'analyse selon l'une des revendications 1 à 5, **caractérisé en ce que** les zones de marquage (12) forment une échelle de déplacement pouvant être explorée de façon incrémentale.

7. Système d'analyse selon l'une des revendications 1 à 6, **caractérisé en ce que** les zones de marquage (12) sont formées par des bandes de trame (22) composées de préférence d'une alternance de segments clairs et foncés.

8. Système d'analyse selon l'une des revendications 1 à 7, **caractérisé en ce que** les zones de marquage (12) comportent deux bandes de trame (22) parallèles l'une à l'autre et décalées l'une par rapport à l'autre dans le sens de la bande.

9. Système d'analyse selon l'une des revendications 1 à 5, **caractérisé en ce que** les zones de marquage (12) comportent une échelle de déplacement à codage absolu.
